# EUROPEAN PATENT SPECIFICATION

(11) **EP 3 020 429 B1**
(45) Date of publication and mention of the grant of the patent: **01.07.2020**
(21) Application number: 15197830.1
(22) Date of filing: 21.06.2010
(51) Int. Cl.: A61M 5/158, A61M 5/00

(54) **DEVICE FOR INSERTING AN INSERTION MEMBER INTO THE TISSUE OF THE BODY OF A PATIENT**
VORRICHTUNG ZUM EINSETZEN EINES EINSATZELEMENTS IN DAS GEWEBE EINES PATIENTENKÖRPERS
DISPOSITIF POUR INSÉRER UN ÉLÉMENT D'INSERTION DANS LE TISSU CORPOREL D'UN PATIENT

(43) Date of publication of application: 18.05.2016
(62) Divisional of application: 10006420.3
(73) Proprietor: F. Hoffmann-La Roche AG, 4070 Basel (CH); Roche Diabetes Care GmbH, 68305 Mannheim (DE)
(72) Inventor: Teutsch, David, 3270 Aarberg (CH); Huwiler, Christoph, 6415 Arth (CH); Michel, Philipp, 3052 Zollikofen (CH)
(74) Representative: Rentsch Partner AG

(56) References cited:
- EP-A1- 1 764 125
- EP-A1- 1 970 091
- WO-A1-2007/140631
- US-A1- 2004 158 207
- US-A1- 2008 269 687
- US-A1- 2008 319 414

## Description

### Field of the invention

The present invention relates to a device for inserting at least one insertion member having a puncturing tip into the tissue of the body of a patient and for fixing it to the skin of the body in accordance with the preamble of the independent claim.

### Background of the invention

For patients with a regular requirement for a medicament that can be administered by direct delivery into the body tissue or into the blood stream, for example certain groups of patients suffering from pain, or patients with type I or type II diabetes, it can be useful to supply the body with the required quantity of medicament in liquid form via a cannula that is introduced at a suitable location into the body and that remains there over quite a long period of time. For this purpose, a cannula arrangement, designated as an infusion set or port, depending on its design, is secured on the patient's skin in such a way that the cannula passes through the skin and into the body.

Efforts are also increasingly being made to monitor certain medical parameters of a patient, for example the blood sugar value, continuously over quite a long period of time. For this purpose, a sensor arrangement, for example, is placed on the patient's body and, with a puncturing tip of a suitable sensor, passes through the skin and into the patient's body.

To avoid infections, the infusion set, the port or the sensor arrangement has to be changed at regular intervals, for example every three days. In outpatient treatment, for example in the case of diabetics, this is often done by the patients themselves and, on account of the introduction of the infusion cannula or the puncturing tip, respectively, into the skin, is associated with a certain amount of pain. It is therefore important that such infusion sets, ports or sensor arrangements can be applied easily and safely, which is why many manufacturers have in the meantime started designing their products as insertion heads for special insertion devices with the aid of which these insertion heads can be applied to the patient's body.

US 2002/0022855 A1 discloses such insertion devices for applying infusion sets having a base plate with skin compatible glue to the body of a patient. With these insertion devices, the infusion sets can be placed onto the application site of the body by the force of a pre-tensioned spring, so that the cannula of the infusion set abruptly penetrates into the tissue of the body and the infusion set with its base plate is fixed to the skin of the body.

US 2008/0319414 discloses an insertion apparatus for use with a device for delivery of a therapeutic fluid into a body of a patient and/or for sensing of a bodily analyte. The apparatus includes a displacement mechanism capable of protracting a penetrating cartridge towards the body, where protraction results in insertion of a subcutaneously insertable element into the body.

US 2004/0158207 discloses a device for inserting a cannula into tissue, including a cannula, a protective element which can accommodate the cannula, an operating element for moving the cannula out of the protective element, and holder fixedly connected to the cannula. The invention encompasses a system for connecting a liquid supply to the cannula.

WO 2007/140631 discloses an infusion set with a cannula protruding from it, and a connection port for the connector of a supply line. The cannula is comprised in a first structural part which is rotatable relative to a second structural part comprising the connection port.

EP 1 970 091 discloses an insertion head having a housing formed by two grip components. A flexible cannula is supported by a puncture device. The device and cannula are in a protected position inside the housing and are deployed by gripping the housing parts and moving one of the parts relative to the other. The device and the cannula are inserted into the body of a patient upon deployment.

EP 1 764 125 discloses an inserter/infusion head system comprising an infusion head with a swiveling needle and an inserter with a casing containing a drive which lowers the infusion head. A telescopic base unit on the casing swivels the needle into the operating position.

US 2008/0269687 discloses patches for medical devices. An adhesive patch of a medical device has selective areas with adhesive material of varying adhesion strengths, or has adhesive material that may be activated by a catalyst to increase or decrease the adhesion strength of the adhesive material. A medical device includes at least one of a needle and a cannula for insertion into the skin of the user during operation of the medical monitoring or treatment device. The at least one of the needle and the cannula are arranged in a first area of a base.

Even though with these known insertion devices the application of infusion sets is made easier and, thanks to the quick and targeted puncturing procedure, the pain associated with the application is significantly reduced compared with a manual application, there is still a potential for further improvement.

A further disadvantage of the known devices is that the fixation of the infusion set to the skin of the body often is unsatisfactory so that, after the application, the infusion set must by hand be pressed against the skin in order to ensure a proper fixation. This, however, is associated with the risk that the cannula might be flexed or be partially pulled out of the tissue and can result in an irritation of the application site.

### Summary of Invention

Therefore, the object of the present invention is to make available a device for inserting an insertion member having a puncturing tip into the tissue of the body of a patient, which do not have, or at least partially avoid, the disadvantages of the prior art.

This object is achieved by the device according to the independent claim.

Accordingly, an aspect of the invention concerns a device for inserting one or several insertion members (of the same or of different kind) having a puncturing tip, like e.g. infusion cannulas and/or sensor tips, into the tissue of the body of a patient and, at the same time, fixing them to the skin of the body. The device comprises a housing, an insertion arrangement and drive means.

The housing provides at least one contact face for placing the device onto the skin of the body of the patient. This contact face carries a layer of skin compatible glue for fixing it to the skin of the body. Such skin compatible glues are known to persons skilled in the art and do not need to be further specified here. Preferably, the layer of skin compatible glue is covered by a removable protecting layer, e.g. a protective film, which before use of the device has to be removed in order to make accessible the glue.

The insertion arrangement is movably arranged within the housing and comprises the one or several insertion members which are to be inserted into the tissue. It furthermore comprises a holder element which is fixedly connected to those part of each of the one or several insertion members which is intended to remain for a longer period inside the tissue. This connection can be established e.g. by bonding or through one piece design.

The drive means are arranged within the housing of the device. They are designed for effecting an insertion movement of the insertion arrangement from a first position, in which the puncturing tips of the one or several insertion members are set back relative to the contact face of the housing, into a second position, in which said puncturing tips protrude beyond the contact face. Thus, when the device during the insertion movement of the insertion arrangement is placed with its contact face onto the skin of the body of a patient, the puncturing tips of the one or several insertion members penetrate through the skin of the body and the insertion members are inserted into the tissue of the body of the patient.

The contact face of the housing is formed by a base plate which is fixed to the housing in such a manner that, when the insertion arrangement after the insertion movement has been performed is in the second position, it is detachable from the rest of the housing. In case of embodiments like e.g. specified further down, in which additional members are temporarily employed in the insertion process which are not intended to remain inside the tissue for a longer period, like e.g. guiding members for stiffening soft members to be inserted into the tissue, the base plate might be detachable from the housing only after these additional members have been removed or retracted, respectively, especially in cases in which the additional member in the non-removed or non-retracted position would impede the detaching. The holder element of the insertion arrangement comprises coupling means by which it can be coupled to corresponding coupling means of the base plate in order to establish a connection between the insertion arrangement and the base plate which allows to safely hold the insertion arrangement by means of the base plate at the body of a patient.

The device is designed in such a manner that the connection between the coupling means of the holder element of the insertion arrangement and the corresponding coupling means of the base plate is automatically established when, by performing the insertion movement, the insertion arrangement is brought into the second position.

With the device according to the invention it becomes possible to fix a base plate, which is used to safely hold the insertion member at the body of the patient, at the desired application site to the skin of the body prior to the insertion of the insertion member into the tissue. This has the advantage that the base plate does not need to be accelerated by the drive means during the insertion movement. Thus, less driving energy is needed for effecting the insertion movement so that the insertion movement can take place with a higher speed, resulting in a further reduction of the pain associated with the piercing of the skin compared to the insertion devices know in the prior art. Furthermore, this offers the advantage that, before the insertion of the insertion member into the tissue, the base plate can properly be fixed to the skin of the body at the application site by pressing the device by hand with the layer of skin compatible glue against the skin of the body, thus, the risk of a flexing of the insertion member or of an irritation of the application site due to a movement of the inserted insertion member caused by an insufficient fixation of the base plate is practically eliminated.

In a preferred embodiment, the device is designed in such a manner that the base plate is automatically separated from the rest of the housing when the insertion arrangement is brought into the second position, thus, the rest of the housing can be removed from the base plate without further handling steps. Through such a design, the handling of the device is further facilitated and the risk of an irritation of the application site is further reduced.

In a further preferred embodiment of the device, at least one of the one or several insertion members of the insertion arrangement comprises, as the part that is intended to remain inside the tissue for a longer period, a soft member and additionally a guiding member associated to this soft member for temporarily stiffening this soft member during its insertion into the tissue. The guiding member forms the puncturing tip for piercing the skin of the body of the patient when inserting this insertion member into the tissue. The soft member is fixedly connected with the holder element. After the insertion movement of the insertion arrangement has been performed, the soft member is coupled to the base plate by means of the coupling means of the holder element and the associated guiding member is coupled to the rest of the housing in such a manner that, when the rest of the housing is removed from the base plate, the guiding member is retained in the rest of the housing and is removed together with the rest of the housing. Preferably, all insertion members of the insertion arrangements comprise a soft member which is intended for remaining for a longer period inside the tissue and an associated guiding member for temporarily stiffening said soft member during insertion into the tissue. Such embodiments have the advantage that an irritation of the application site during the application period is kept at a minimum, since the parts of the insertion members which stay in the tissue of the body are soft elements. i.e. are elements which have a certain flexibility so that they can follow movements of the body tissue.

In embodiments in which at least one of the one or several insertion members comprises a soft member which is intended for remaining for a longer period inside the tissue and an associated guiding member for temporarily stiffening said soft member during insertion into the tissue, it is furthermore preferred that the device further comprises drive means for effecting, after the insertion movement of the insertion arrangement, a retracting movement of the guiding member from the second position into a third position. In this third position, the puncturing tip of the insertion member comprising the soft member and the associated guiding member is set back relative to the contact face of the housing, so that, when during the retracting movement the soft member is held in the second position by the holder element coupled to the base plate, the guiding member is separated from the soft member.

Preferably, the device in that case is designed in such a manner that, after the insertion movement of the insertion arrangement is performed, automatically the retracting movement of the at least one guiding member is performed.

With such devices, the insertion of a soft member, like e.g. a soft cannula, into the tissue of the body of a patient is easy and safe even for unskilled persons, since the risk of an irritation of the puncture site due to an unskilled removal of the guiding member as well as the risk of an unintended contact with the contaminated guiding member or even of an injury caused by a contact with the puncturing tip is virtually eliminated.

Furthermore is preferred in the two before mentioned cases that the device is designed in such a manner that, when the guiding member after the retracting movement is arranged in the rest of the housing in the third position and the rest of the housing has been removed from the soft member, the guiding member cannot be separated from the rest of the housing or can be separated from the rest of the housing without contacting it or a holder element fixedly connected to it. The first alternative is preferred if the housing is designed as a disposable part so that the contaminated guiding member can be disposed together with the rest of the housing. The second alternative is preferred if the rest of the housing is intended to be used several times and the guiding member for disposal is separated from the housing. In both cases the advantage is arrived at that for disposal of the contaminated guiding member only the housing needs to be contacted by hand, thus, the risk of a contact with the contaminated guiding member and in particular with its puncturing tip is further reduced.

Also it is furthermore preferred in the before mentioned embodiments of the device, in which at least one of the one or several insertion members of the insertion arrangement comprises a soft member and an associated guiding member, that the soft member is a soft cannula and the associated guiding member is a guiding needle which extends through the fluid channel of said soft cannula for temporarily stiffening it during introduction into the tissue. In that case in embodiments in which the guiding needle is retracted into a third position, the device is designed in such a manner that, when the retracting movement of the at least one guiding needle is performed while the soft cannula is held in the second position by the holder element coupled to the base plate, the guiding needle is pulled out of the fluid channel of the at least one soft cannula. In such embodiments, the advantages of the invention become particularly apparent.

In still a further preferred embodiment, the device further comprises activating means for activating the drive means. The activating means are designed in such a manner that they can be actuated by the user with one hand. This considerably facilitates the operation of the device and furthermore permits its use for the application of insertion members in body regions which are difficult to access or which are not accessible with both hands.

According to the invention, the drive means of the device comprise at least one energy source for providing driving energy for the insertion movement and/or, where applicable, for the retracting movement, like e.g. a pre-tensioned spring element, a pressurized gas reservoir, a battery, an accumulator and/or a pyrotechnical propelling charge.

The drive means comprise at least one energy storing element for providing driving energy for the insertion movement and/or, where applicable, for the retracting movement, which, before use of the device, must be charged with energy. Preferably, said at least one energy storing element comprises a spring element that can be pre-tensioned and/or a gas reservoir that can be pressurized. Such devices are comfortable in use and allow an insertion movement with defined insertion speed and insertion force.

The device further comprises manual energy charging means, which can be actuated by hand to transfer power generated by the muscles of the hand of a user to the at least one energy storing element. Preferably, these manual energy charging means are designed in such a manner that they can be actuated with one hand. Such embodiments have the advantage that they may be independent from foreign energy sources and, if so, can be operated everywhere and at any time.

According to the invention, said manual energy charging means comprise two housing elements that can be pushed into or towards each other. The direction of sliding along the housing, pushing into the housing or pushing into or towards each other preferably is transverse, in particular perpendicular, to the direction of the insertion movement of the insertion arrangement. This considerably facilitates the integration of the manual energy charging means into the device and the operation of the device by the user.

In embodiments, in which the device comprises activating means which can be actuated by the user with one hand and furthermore comprises at least one energy storing element and manual energy charging means for charging said energy storing element, it is preferred that the activating means and the manual energy charging means are designed in such a manner that charging of the at least one energy storing element and activating of the driving means for effecting the insertion movement is effected through the same manual operation.

In embodiments of the device which are designed to perform a retracting movement of one or several guiding members of the insertion arrangement, the drive means for effecting the insertion movement can be the same or can be different drive means than the drive means for effecting the retracting movement. Furthermore, the energy for the insertion movement and the energy for the retraction movement can commonly or separately be provided by one or by several energy sources of the same kind or of different kind, in particular by a combination of hand energy and pressurized gas and/or pre-loaded springs. For example, a pre-loaded spring might provide the insertion energy while the hand provides the retraction energy.

In still a further preferred embodiment of the device, the insertion arrangement in the first position is oriented substantially perpendicular to a plane formed by the at least one contact face of the housing and the insertion movement is a linear movement along an axis perpendicular to said plane. By means of this, the insertion member can be inserted into the tissue of the body of a patient perpendicular to the surface of the skin of the body, which in most of the applications is preferred.

In the before mentioned case it is furthermore preferred that, in an initial position, the insertion arrangement is oriented substantially parallel to said plane formed by the at least one contact face of the housing and, before the insertion movement can be performed, must be pivoted from said initial position into the first position. By this is becomes possible to provide devices according to the invention which in the original non-used state have small packaging dimensions.

If additionally the device further comprises activating means for activating the drive means, it is preferred that the device is designed in such a manner that, upon an actuating of the activating means, the insertion arrangement is automatically moved from the initial position into the first position. This facilitates the use of the device.

In still a further preferred embodiment, the device is formed by an inserting device which provides the housing without base plate and the drive means, by an insertion head, in particular an infusion set, which provides the insertion arrangement and by a base plate, which can be part of the insertion head or can be separate. The insertion head is received within the inserting device for being applied by it to the body of the patient and the base plate is fixed to the housing in order to be pressed with the housing against the skin of the body of a patient. The inserting device is designed in such a manner that, after the application of the insertion head, it separation from the base plate and, where applicable, the removal of additional members of the insertion head which have been retained in it, like e.g. guiding members of the applied insertion head, it can receive another insertion head for applying it to the body of a patient. By means of this, the amount of waste generated in therapies employing the frequent insertion of insertion members into the body of a patient can be kept at a minimum.

Preferably, the device according to the invention is used for applying an infusion set to the body of a patient, preferably an infusion set having a soft cannula.

Another aspect of the disclosure concerns a method of inserting one or several insertion members having a puncturing tip, in particular having at least one infusion cannula and/or at least one sensor tip, into the tissue of the body of a patient and of fixing it to the skin of said body, both by using the device according to the invention. In a first step, the device is placed with its layer of skin compatible glue onto the skin of the body at the desired application site for fixing the base plate to the body. In a second step, the drive means of the device are activated, thereby effecting the insertion movement of the at least one insertion member into the tissue of the body and the coupling of the one or several insertion members to the base plate. In a third step, the rest of the housing is separated from the base plate while the parts of the insertion members which are intended to remain for a longer period within the tissue remain inserted in the tissue of the body of the patient and are fixed to the skin of the body by the base plate.

With the method according to the disclosure, the application of an infusion set to the body of a patient can be performed, even by unskilled or hampered persons, in an extremely safe and easy manner, without the risk of a flexing of the insertion member or of an irritation of the application site due to a movement of the inserted insertion member caused by an insufficient fixation of the base plate. Furthermore, the insertion movement can take place with a higher speed compared to methods known from the prior art, resulting in a further reduction of the pain associated with the piercing of the skin.

In a preferred embodiment of the method, the insertion member used in the method is a soft cannula and the guiding member used is a guiding needle which, at the time the soft cannula is inserted into the tissue, extends through the fluid channel of the soft cannula.

### Brief description of the drawings

Further preferred embodiments of the invention become apparent from the dependent claims and from the following description by way of the drawings.
Figure 1a shows a perspective view onto a device according to the invention in a first state.
Figure 1b shows a perspective longitudinal section of the device of Figure 1a.
Figure 2a shows a perspective view of the device of Figure 1a in a second state.
Figure 2b shows a perspective longitudinal section of the device of Figure 2a.
Figure 3a shows a perspective view of the device of Figure 1a in a third state.
Figure 3b shows a perspective longitudinal section of the device of Figure 3a.
Figure 4a shows a perspective view of the device of Figure 1a in a fourth state.
Figure 4b shows a perspective longitudinal section of the device of Figure 4a.
Figure 5a shows a perspective view of the device of Figure 1a in a fifth state.
Figure 5b shows a perspective longitudinal section of the device of Figure 5a.
Figure 5c shows a cross section of the device of Figure 5a.
Figure 6 shows a perspective longitudinal section of the device of Figure 1a in a sixth state.
Figure 7 shows a perspective longitudinal section of the device of Figure 1a in a seventh state.

### Detailed description of the invention

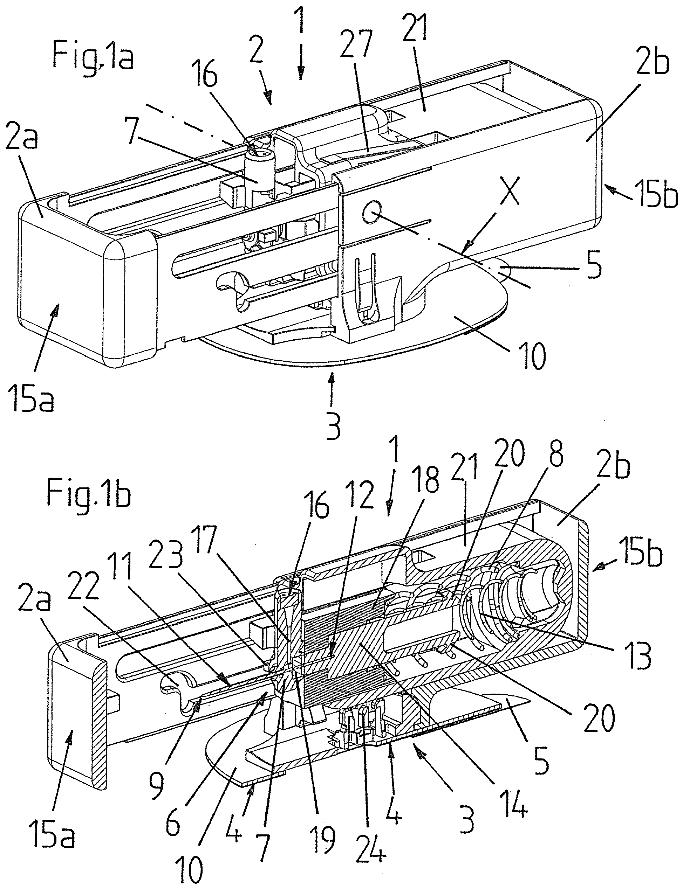

The Figures 1a and 1b show a device 1 according to the invention in a first state (original state), once in a perspective side view (Figure 1a) and once in a perspective longitudinal section (Figure 1 b). As can be seen here, the device 1 comprises a housing 2 which substantially is formed by two housing elements 2a, 2b which for operating the device can be pushed into each other and a drive unit housing 21 which in the situation shown in these figures is arranged within the second housing element 2b. On its bottom side, the second housing element 2b carries a base plate 10 providing a contact face 3 for placing the device 1 onto the skin of the body of a patient. This contact face 3 carries a layer 4 of skin compatible glue for fixing the base plate 10 to the skin of the body, which layer 4 is covered by a removable protecting film 5 which before use of the device 1 must be removed.

Within the housing 2, 2a, 2b, a cannula arrangement 6 is arranged in a movable manner, which comprises a soft cannula 11 for being inserted into the tissue of the body of the patient and a guiding needle 12 extending through the fluid channel of said soft cannula 11 for temporarily stiffening it during introduction into the tissue. The soft cannula 11 together with the guiding needle 12 forms an insertion member 11, 12 according to the claims, wherein the soft cannula is the part of this insertion member that is intended to remain inside the tissue of the body for a longer period. The guiding needle 12 at the free end of the cannula arrangement 6 protrudes out of the soft cannula 11, thereby forming a puncturing tip 9 for puncturing the skin of the body of the patient when inserting the soft cannula 11 with the help of the guiding needle 12 into the tissue. The soft cannula 11 at its end facing away from the puncturing tip 9 is fixedly connected through material bonding to an adapter housing 7 (holder element 7 according to the claims) providing a port 16 for connecting a catheter to the housing 7 and a fluid channel 17 extending inside the housing 7 between the soft cannula 11 and the port 16, for fluidically connecting the catheter with the soft cannula 11. The guiding needle 12 at its end facing away from its puncturing tip 9 protrudes through a septum 19 provided by the adapter housing 7 into a holder element 14 to which it is fixedly connected. The holder element 14 is in a slidable manner received in a guiding sleeve 18 which in turn in a slidable manner is guided inside the drive unit housing 21 which in a tiltable manner around an axis X is arranged inside the second housing element 2b. The soft cannula 11 and the adapter housing 7 are carried by the guiding needle 12. In this state, the cannula arrangement 6 is oriented substantially parallel to a plane formed by the contact face 3.

The device 1 furthermore comprises helical springs 8, 13 arranged in a coaxial manner within the drive unit housing 21, the function of which will in the course of the proceeding description of the drawings be described more into detail.

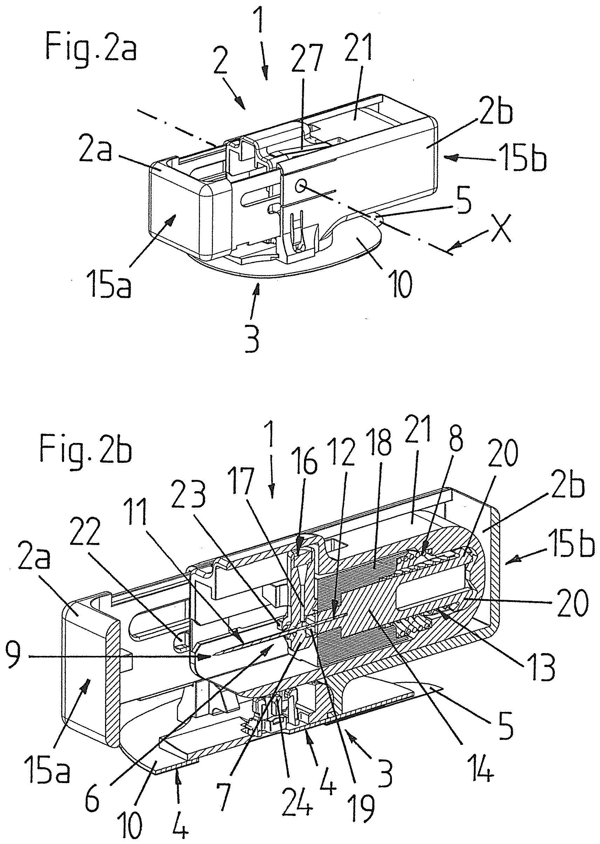

For operating the device 1, the front faces 1 5a, 15b (activating means 1 5a, 1 5b according to the claims) of the two housing elements 2a, 2b are contacted with the fingertips of the trigger finger and thumb of one hand of the operator and are pressed towards each other. By doing so, the two housing elements 2a, 2b are pushed into each other and the guiding sleeve 18, which carries the cannula arrangement 6, is pushed by the first housing element 2a into the drive unit housing 21 against the force of the two springs 8, 13, which both with one of their two ends abut against the guiding sleeve 18 and with their other end abut against an end of the drive unit housing 21. During this movement of the guiding sleeve 18, the holder element 14 travels through the centers of the springs 8, 13 and at the end, when both springs 8, 13 are completely pre-tensioned, snaps with resilient hook elements 20 formed at its end behind the end of the inner spring 13 which abuts against the end of the drive unit housing 21. At the same time, latching rockers 27 arranged at the drive unit housing 21 snap behind lugs 28 arranged at the guiding sleeve 18, thereby arresting the guiding sleeve 18 in a state in which the springs 8, 13 are pre-tensioned. This situation, in which the first housing element 2a has been pushed by about two thirds of its possible travel into the second housing element 2b, is shown in the Figures 2a and 2b, once in a perspective side view (Figure 2a) and once in a perspective longitudinal section (Figure 2b).

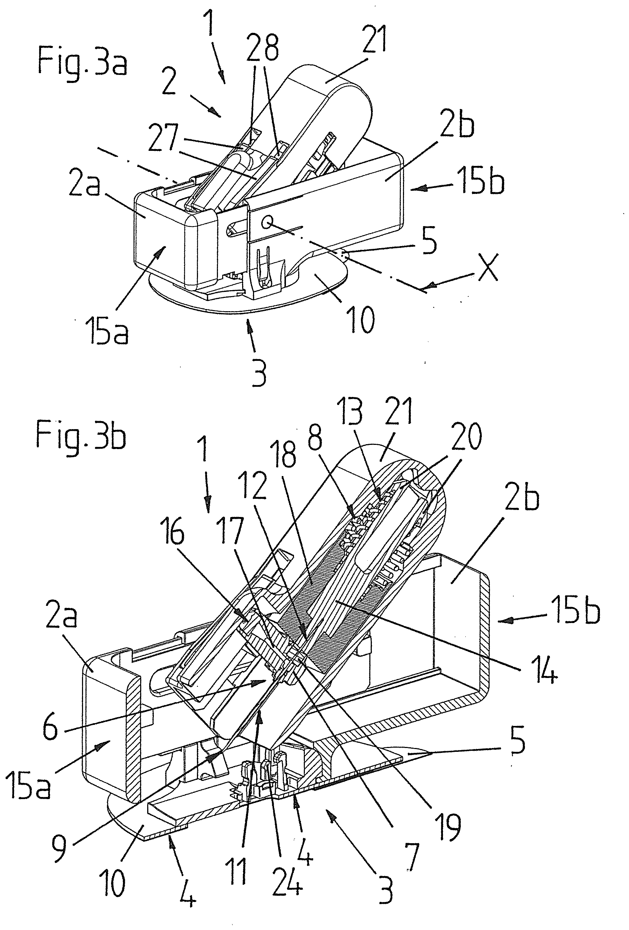

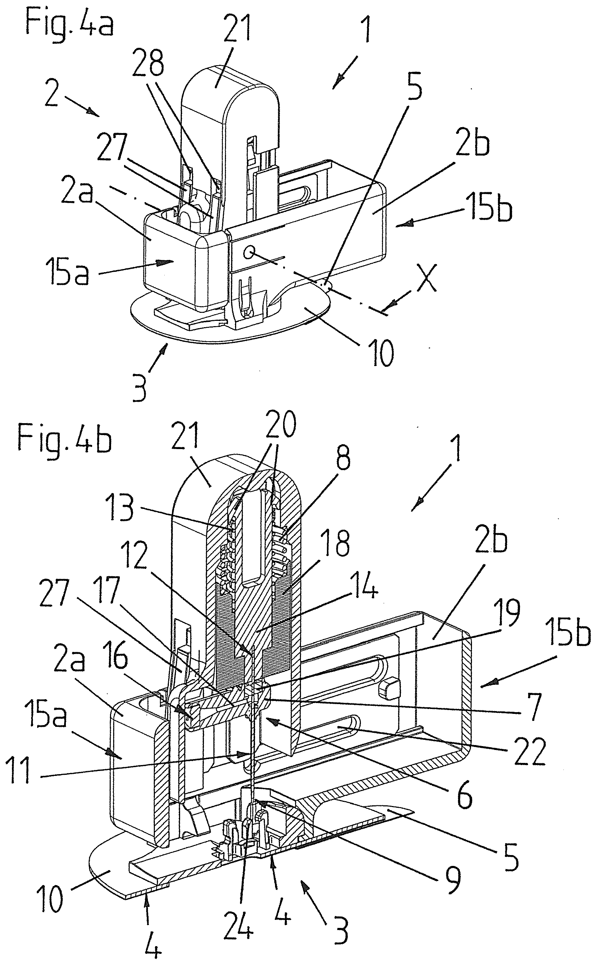

By further pushing the two housing elements 2a, 2b inside each other, the drive unit housing 21 with the cannula arrangement 6 carried by it is tilted around its tilting axis X from its initial position, in which the cannula arrangement 6 is oriented parallel to a plane formed by the contact face 3, over an intermediate position shown in the Figures 3a and 3b into its operating position (first position according to the claims) shown in the Figures 4a and 4b, in which the cannula arrangement 6 is oriented perpendicular to the plane formed by the contact face 3. In this situation, the puncturing tip 9 of the guiding needle 12 is set back relative to the contact face 3. The tilting movement of the drive unit housing 21 is effected by a pivot formed at the drive unit housing 21 which travels in a guiding slot 22 in the first housing element 2a. As is visible especially in Figure 4a, in this state the housing elements 2a, 2b are almost fully pushed towards each other.

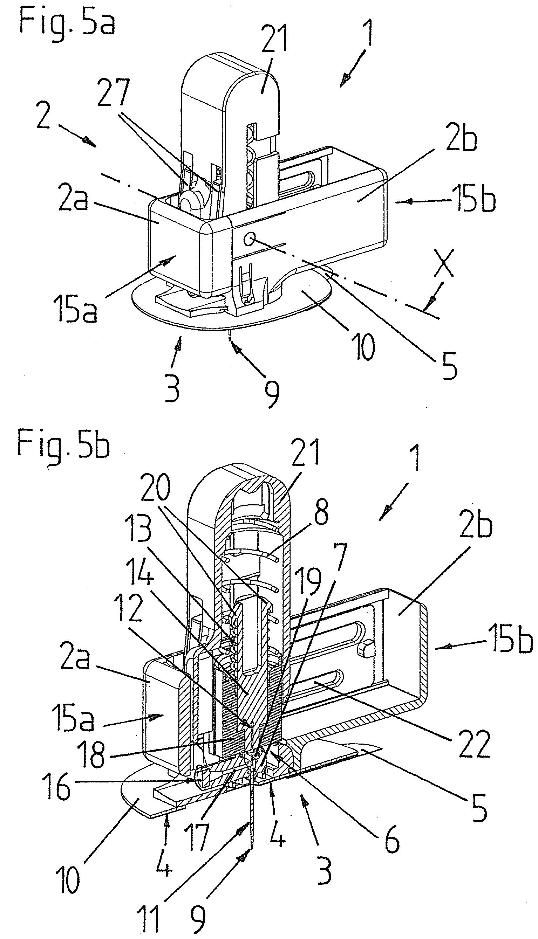

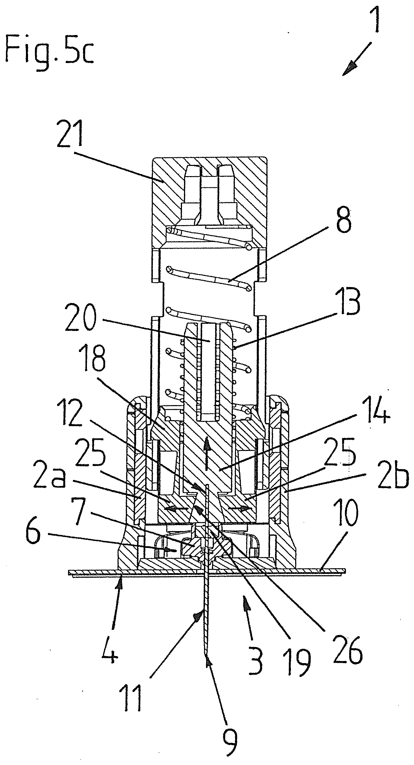

By completely pushing the two housing elements 2a, 2b towards each other, the latching rockers 27 are actuated, thereby releasing the lugs 28. The force of the pre-tensioned outer spring 8 now drives the guiding sleeve 18 and with it the cannula arrangement 6 in a direction perpendicular to the plane formed by the contact face 3 towards the base plate 10, where at the end of this movement (the insertion movement according to the claims) the adapter housing 7 with protrusions 23 formed at its outside snaps behind resilient hook elements 24 formed at the base plate 10 and thereby in a positive manner is coupled to the base plate 10. In this position (second position according to the claims), which is shown in the Figures 5a, 5b and 5c, the puncturing tip 9 and large portions of the guiding needle 12 as well as of the soft cannula 11 protrude beyond the contact face 3 of the base plate 10.

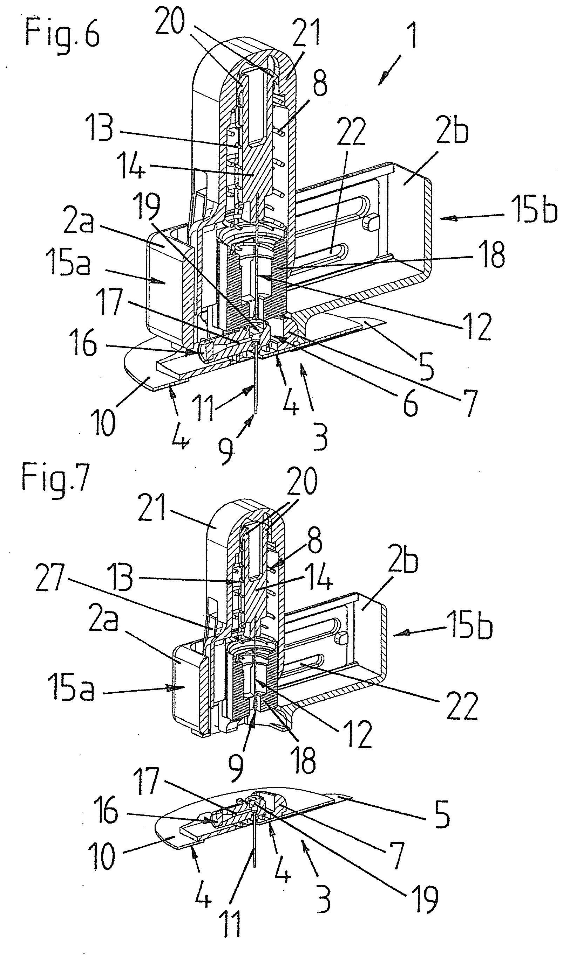

As can be seen in Figure 5c, which shows a cross section of the device 1 in this state, the guiding sleeve 18 comprises two resilient hook elements 25, which abut with contact surfaces 26 that are inclined relative to the direction of the insertion movement against corresponding contact surfaces formed at the holder element 14 of the guiding needle 12. During the insertion movement, the hook elements 25 are radially blocked by guiding surfaces of the drive unit housing 21, so that during the insertion movement, the holder element 14 in a positive manner in movement direction is coupled to the guiding sleeve 18. In the end position of the insertion movement shown in the Figures 5a to 5c, the hook elements 25 are free to radially expand. In this situation, the holder element 14 is pulled upwards through the force of the inner spring 13, thereby radially expanding the hook elements 25 by its inclined contact surfaces (see the arrows in Figure 5c) and pulling the guiding needle 12 out of the soft cannula 11 and out of the septum 19 of the adapter housing 7 (retracting movement according to the claims) into a position in which its puncturing tip 9 is set back relative to the contact face 3 (third position according to the claims). In this situation, which is shown in Figure 6, the second housing element 2b can be detached from the base plate 10 and can, together with the guiding needle 12 contained therein, be removed from the base plated 10 without any risk that its puncturing tip can be contacted by hand and can cause an injury to persons. This situation is shown in Figure 7.

Certain particularly advantageous examples of the disclosed devices for inserting an insertion member are provided in the following:
A first advantageous embodiment of a device (1) for inserting at least one insertion member (11, 12) having a puncturing tip (9), in particular at least one infusion cannula and/or at least one sensor tip, into the tissue of the body of a patient and for fixing it to the skin of the body, comprises: a) a housing (2, 2a, 2b, 10, 21) providing at least one contact face (3) for placing the device (1) onto the skin of the body of the patient, wherein the contact face (3) carries a layer (4) of skin compatible glue for fixing it to the skin, which layer (4) in particular is covered by a removable protecting layer (5), b) an insertion arrangement (6) movably arranged within the housing (2, 2a, 2b, 10, 21) comprising the at least one insertion member (11, 12) to be inserted into the tissue and a holder element (7) fixedly connected with at least a part (11) of the at least one insertion member (11, 12) which is intended to remain for a longer period inside the tissue of the body, and c) drive means (8) arranged within the housing (2, 2a, 2b, 10, 21) for effecting an insertion movement of the insertion arrangement (6) from a first position, in which the puncturing tip (9) of the at least one insertion member (11, 12) is set back relative to the contact face (3), into a second position, in which the puncturing tip (9) of the at least one insertion member (11, 12) protrudes beyond the contact face (3), so that the at least one insertion member (11, 12) is inserted into the tissue of the body of the patient when the device (1) during the insertion movement of the insertion arrangement (6) is placed with its contact face (3) on said body, wherein the contact face (3) is formed by a base plate (10) which is fixed in such a manner that, after the insertion movement has been performed, it is detachable from the rest of the housing (2, 2a, 2b, 21) and wherein the holder element (7) of the insertion arrangement (6) comprises coupling means (23) by which it is coupled to the base plate (10) when the insertion arrangement (6) by the insertion movement is brought into the second position.

In an advantageous variant of such a device (1), the device (1) is designed in such a manner that the base plate (10) is automatically separated from the rest of the housing (2, 2a, 2b, 21) when the insertion arrangement (6) is brought into the second position.

In a further advantageous variant of said devices (1), the at least one insertion member (11, 12) of the insertion arrangement (6) comprises at least one soft member (11) for being inserted into the tissue and at least one guiding member (12) for temporarily stiffening the at least one soft member (11) during insertion into the tissue, which guiding member (12) forms the puncturing tip (9) for puncturing the skin of the body of the patient when inserting the at least one insertion member (11, 12) into the tissue. The holder element (7) is fixedly connected with the at least one soft member (11). When after the insertion movement of the insertion arrangement (6) the at least one soft member (11) is coupled to the base plate (10) by means of the coupling means of the holder element (7), the at least one guiding member (12) is coupled to the rest of the housing (2, 2a, 2b, 21) in such a manner that, when the rest of the housing (2, 2a, 2b, 21) is removed from the base plate (10), the at least one guiding member (12) is retained in the rest of the housing (2, 2a, 2b, 21) and is removed together with the rest of the housing (2, 2a, 2b, 21). Such an advantageous device (1) may further comprise drive means (13) for effecting, after the insertion movement of the insertion arrangement (6), a retracting movement of the at least one guiding member (12) from the second position into a third position, in which its puncturing tip (9) is set back relative to the contact face (3), so that, when the at least one soft member (11) is held in the second position by the holder element (7) coupled to the base plate (10), the at least one guiding member (12) is separated from the at least one soft member (11). Even more advantageous, the device (1) may be designed in such a manner that, after the insertion movement of the insertion arrangement (6) is performed, automatically the retracting movement of the at least one guiding member (12) is performed. Furthermore, the device (1) may be designed in such a manner that, when the at least one guiding member (12) after the retracting movement is arranged in the rest of the housing (2, 2a, 2b, 21) in the third position and the rest of the housing (2, 2a, 2b, 21) has been removed from the at least one soft member (11), the at least one guiding member (12) can not be separated from the rest of the housing (2, 2a, 2b, 21) or can be separated from the rest of the housing (2, 2a, 2b, 21) without contacting it or a holder element (14) fixedly connected with it.

In such a device (1), the at least one soft member (11) may comprise at least one soft cannula (11), and the at least one guiding member (12) may comprise at least one guiding needle (12), which extends through the fluid channel of said soft cannula (11) for temporarily stiffening it during introduction into the tissue.

The above-mentioned advantageous embodiments of devices (1) may further comprise activating means (15a, 15b) for activating the drive means (8, 13), wherein the activating means (15a, 1 5b) are designed in such a manner that they can be actuated by the user with one hand.

In the above-mentioned advantageous embodiments of devices (1), advantageously in the first position the insertion arrangement (6) is oriented substantially perpendicular to a plane formed by the at least one contact face (3) and the insertion movement is a linear movement along an axis perpendicular to said plane. More advantageously, in an initial position the insertion arrangement (6) is oriented substantially parallel to a plane formed by the at least one contact face (3) and before the insertion movement can be performed must be pivoted from the initial position into the first position.

The above-mentioned advantageous embodiments of devices (1) may comprise activating means (15a, 15b) for activating the drive means (8, 13), wherein the activating means (15a, 1 5b) are designed in such a manner that they can be actuated by the user with one hand, wherein in an initial position the insertion arrangement (6) is oriented substantially parallel to a plane formed by the at least one contact face (3) and before the insertion movement can be performed must be pivoted from the initial position into the first position, and wherein the device (1) is designed in such a manner that upon an actuating of the activating means (15a, 15b), the insertion arrangement (6) is automatically moved from the initial position into the first position.

In the above-mentioned advantageous embodiments of devices (1), advantageously the device (1) is formed by an inserting device providing the housing and the drive means, by an insertion head providing the insertion arrangement and by a base plate provided by the insertion head or provided separately, wherein the insertion head is received within the inserting device for being applied to the body of the patient by means of said inserting device and the base plate is fixed to the housing in order to be pressed with the housing against the skin of the body of a patient, and wherein the inserting device is designed in such a manner that, after the application of the insertion head and its removal from the base plate, it can receive another insertion head for applying it to the body of a patient.

An advantageous method of inserting at least one insertion member (11, 12) having a puncturing tip (9), in particular having at least one infusion cannula and/or at least one sensor tip, into the tissue of the body of a patient and of fixing it to the skin of the body by using an above-mentioned device (1), comprises the following steps: a) placing the device (1) with the layer (4) of skin compatible glue onto the skin of the body at the desired application site for fixing the base plate (10) to the body; thereafter b) activating the drive means (8, 13), thereby effecting the insertion movement of the at least one insertion member (11, 12) into the tissue of the body and the coupling of at least a part (11) of the at least one insertion member (11, 12) which is intended to remain for a longer period inside the tissue of the body to the base plate (10); and thereafter c) separating the rest of the housing (2, 2a, 2b, 21) from the base plate (10) and from the part (11) of the at least one insertion member (11, 12) coupled thereto, thereby leaving the part (11) of the at least one insertion member (11, 12) which is intended to remain for a longer period inside the tissue inserted in the tissue of the body of the patient and fixed to the skin of said body by the base plate (3).

While there are shown and described presently preferred embodiments of the invention, it is to be distinctly understood that the invention is not limited thereto but may be otherwise variously embodied and practiced within the scope of the following claims.

## Claims

1. Device (1) for inserting at least one insertion member (11, 12) having a puncturing tip (9) in the form of at least one sensor tip or at least one infusion cannula, into the tissue of the body of a patient and for fixing it to the skin of the body, comprising:
- a housing (2, 2a, 2b, 10, 21) providing at least one contact face (3) for placing the device (1) onto the skin of the body of the patient, wherein the contact face (3) carries a layer (4) of skin compatible glue for fixing it to the skin, which layer (4) in particular is covered by a removable protecting layer (5);
- an insertion arrangement (6) movably arranged within the housing (2, 2a, 2b, 10, 21) comprising the at least one insertion member (11, 12) to be inserted into the tissue and a holder element (7) fixedly connected with at least a part (11) of the at least one insertion member (11, 12) which is intended to remain for a longer period inside the tissue of the body; and
- drive means (8) arranged within the housing (2, 2a, 2b, 10, 21) for effecting an insertion movement of the insertion arrangement (6) from a first position, in which the puncturing tip (9) of the at least one insertion member (11, 12) is set back relative to the contact face (3), into a second position, in which the puncturing tip (9) of the at least one insertion member (11, 12) protrudes beyond the contact face (3), so that the at least one insertion member (11, 12) is inserted into the tissue of the body of the patient when the device (1) during the insertion movement of the insertion arrangement (6) is placed with its contact face (3) on said body;
wherein
- the contact face (3) is formed by a base plate (10) which is fixed in such a manner that, after the insertion movement has been performed, it is detachable from the rest of the housing (2, 2a, 2b, 21);
- the holder element (7) of the insertion arrangement (6) comprises coupling means (23) by which it is coupled to the base plate (10) when the insertion arrangement (6) by the insertion movement is brought into the second position
**characterized in that**
- the drive means comprise at least one energy storing element for providing driving energy for the insertion movement, and
- manual energy charging means for transferring power to the at least one energy storing element comprise two housing elements that can be pushed into or towards each other.

2. Device (1) according to claim 1, wherein the at least one insertion member (11, 12) of the insertion arrangement (6) comprises at least one soft member (11) for being inserted into the tissue and at least one guiding member (12) for temporarily stiffening the at least one soft member (11) during insertion into the tissue, which guiding member (12) forms the puncturing tip (9) for puncturing the skin of the body of the patient when inserting the at least one insertion member (11, 12) into the tissue;
wherein
- the holder element (7) is fixedly connected with the at least one soft member (11); and
- when after the insertion movement of the insertion arrangement (6) the at least one soft member (11) is coupled to the base plate (10) by means of the coupling means of the holder element (7), the at least one guiding member (12) is coupled to the rest of the housing (2, 2a, 2b, 21) in such a manner that, when the rest of the housing (2, 2a, 2b, 21) is removed from the base plate (10), the at least one guiding member (12) is retained in the rest of the housing (2, 2a, 2b, 21) and is removed together with the rest of the housing (2, 2a, 2b, 21).

3. Device (1) according to claim 2, wherein the device (1) further comprises drive means (13) for effecting, after the insertion movement of the insertion arrangement (6), a retracting movement of the at least one guiding member (12) from the second position into a third position, in which its puncturing tip (9) is set back relative to the contact face (3), so that, when the at least one soft member (11) is held in the second position by the holder element (7) coupled to the base plate (10), the at least one guiding member (12) is separated from the at least one soft member (11).

4. Device (1) according to claim 3, wherein the device (1) is designed in such a manner that, after the insertion movement of the insertion arrangement (6) is performed, automatically the retracting movement of the at least one guiding member (12) is performed.

5. Device (1) according to claim 3 or 4, wherein the device (1) is designed in such a manner that, when the at least one guiding member (12) after the retracting movement is arranged in the rest of the housing (2, 2a, 2b, 21) in the third position and the rest of the housing (2, 2a, 2b, 21) has been removed from the at least one soft member (11), the at least one guiding member (12) cannot be separated from the rest of the housing (2, 2a, 2b, 21) or can be separated from the rest of the housing (2, 2a, 2b, 21) without contacting it or a holder element (14) fixedly connected with it.

6. Device (1) according to any of claims 2 to 5, wherein the at least one soft member (11) comprises at least one soft cannula (11) and the at least one guiding member (12) comprises at least one guiding needle (12), which extends through the fluid channel of said soft cannula (11) for temporarily stiffening it during introduction into the tissue.

7. Device (1) according to any of the preceding claims, wherein the device (1) further comprises activating means (15a, 15b) for activating the drive means (8, 13) and wherein the activating means (15a, 15b) are designed in such a manner that they can be actuated by the user with one hand.

8. Device (1) according to any of the preceding claims, wherein in the first position the insertion arrangement (6) is oriented substantially perpendicular to a plane formed by the at least one contact face (3) and the insertion movement is a linear movement along an axis perpendicular to said plane.

9. Device (1) according to claim 8, wherein in an initial position the insertion arrangement (6) is oriented substantially parallel to a plane formed by the at least one contact face (3) and before the insertion movement can be performed must be pivoted from the initial position into the first position.

10. Device (1) according to claim 7 and according to claim 9, wherein the device (1) is designed in such a manner that upon an actuating of the activating means (1 5a, 1 5b), the insertion arrangement (6) is automatically moved from the initial position into the first position.

11. Device (1) according to any of the preceding claims, wherein the device (1) is formed by an inserting device providing the housing and the drive means, by an insertion head providing the insertion arrangement and by a base plate provided by the insertion head or provided separately, wherein the insertion head is received within the inserting device for being applied to the body of the patient by means of said inserting device and the base plate is fixed to the housing in order to be pressed with the housing against the skin of the body of a patient, and wherein the inserting device is designed in such a manner that, after the application of the insertion head and its removal from the base plate, it can receive another insertion head for applying it to the body of a patient.

## Patentansprüche

1. Vorrichtung (1) zum Einführen mindestens eines Einführelements (11, 12) mit einer Punktionsspitze (9) in Form von mindestens einer Sensorspitze oder mindestens einer Infusionskanüle in das Gewebe des Körpers eines Patienten und für Befestigen an der Haut des Körpers, umfassend:
- ein Gehäuse (2, 2a, 2b, 10, 21), das mindestens eine Kontaktfläche (3) zum Aufbringen der Vorrichtung (1) auf die Haut des Körpers des Patienten vorsieht, wobei die Kontaktfläche (3) eine Schicht (4) aus hautverträglichem Klebstoff trägt, um sie an der Haut zu befestigen, wobei diese Schicht (4) insbesondere von einer entfernbaren Schutzschicht (5) bedeckt ist;
- eine Einführanordnung (6), die beweglich in dem Gehäuse (2, 2a, 2b, 10, 21) angeordnet ist, umfassend das mindestens eine Einführelement (11, 12), das in das Gewebe eingeführt werden soll, und ein (7) mit mindestens einem Teil (11) des mindestens einen Einführelements (11, 12), der für einen längeren Zeitraum im Gewebe des Körpers verbleiben soll, fest verbundenes Halteelement; und
- Antriebsmittel (8), die innerhalb des Gehäuses (2, 2a, 2b, 10, 21) angeordnet sind, um eine Einführbewegung der Einführanordnung (6) von einer ersten Position aus zu bewirken, in der sich die Einstichspitze (9) des mindestens einen Einführelements (11, 12) relativ zur Kontaktfläche (3) in eine zweite Position zurückgesetzt ist, in der die Einstichspitze (9) des mindestens einen Einführelements (11, 12) über die Kontaktfläche (3) hinausragt, so dass das mindestens eine Einführelement (11, 12) in das Gewebe des Körpers des Patienten eingeführt wird, wenn die Vorrichtung (1) während der Einführbewegung der Einführanordnung (6) mit der Kontaktoberfläche (3) auf dem Körper angeordnet wird;
wobei
- die Kontaktfläche (3) durch eine Grundplatte (10) gebildet wird, die derart fixiert ist, dass sie nach Durchführung der Einführbewegung vom Rest des Gehäuses (2, 2a, 2b, 21) abnehmbar ist;
- das Halteelement (7) der Einführanordnung (6) Kopplungsmittel (23) umfasst, durch die es mit der Grundplatte (10) gekoppelt ist, wenn die Einführanordnung (6) durch die Einführbewegung in die zweite Position gebracht wird,
**dadurch gekennzeichnet, dass**
- das Antriebsmittel mindestens ein Energiespeicherelement zum Bereitstellen von Antriebsenergie für die Einführbewegung umfasst, und
- manuelle Energielademittel zum Übertragen von Energie auf das mindestens eine Energiespeicherelement umfassend zwei Gehäuseelemente, die ineinander oder aufeinander zu gedrückt werden können.

2. Vorrichtung (1) nach Anspruch 1, wobei das mindestens eine Einführelement (11, 12) der Einführanordnung (6) mindestens ein weiches Element (11) zum Einführen in das Gewebe und mindestens ein Führungselement (12) zum zeitweiligen Versteifen des mindestens einen weichen Elements (11) während des Einführens in das Gewebe umfasst, wobei das Führungselement (12) die Punktionsspitze (9) zum Einstechen der Haut des Körpers des Patienten beim Einführen des mindestens einen Einführelements (11, 12) in das Gewebe bildet;
wobei
- das Halteelement (7) fest mit dem mindestens einen weichen Element (11) verbunden ist; und
- wenn nach der Einführbewegung der Einführanordnung (6) das mindestens eine weiche Element (11) mittels der Kopplungseinrichtung des Halterelements (7) mit der Grundplatte (10) verbunden ist, das mindestens eine Führungselement (12) mit dem Rest des Gehäuses (2, 2a, 2b, 21) derart verbunden ist, dass, wenn der Rest des Gehäuses (2, 2a, 2b, 21) von der Grundplatte (10) entfernt wird, das mindestens eine Führungselement (12) im Rest des Gehäuses (2, 2a, 2b, 21) zurückgehalten und zusammen mit dem Rest des Gehäuses (2, 2a, 2b, 21) entfernt wird.

3. Vorrichtung (1) nach Anspruch 2, wobei die Vorrichtung (1) ferner Antriebsmittel (13) umfasst, um nach der Einführbewegung der Einführanordnung (6) eine Rückzugsbewegung des mindestens einen Führungselements (12) von der zweiten Position in eine dritte Position zu bewirken, in der seine Punktierungsspitze (9) relativ zur Kontaktfläche (3) zurückgesetzt ist, so dass, wenn das mindestens eine weiche Element (11) durch das mit der Grundplatte (10) gekoppelte Halteelement (7) in der zweiten Position gehalten wird, das mindestens eine Führungselement (12) von dem mindestens einen weichen Element (11) getrennt wird.

4. Vorrichtung (1) nach Anspruch 3, wobei die Vorrichtung (1) derart ausgelegt ist, dass nach Durchführung der Einführbewegung der Einführanordnung (6) automatisch die Rückzugsbewegung des mindestens einen Führungselements (12) durchgeführt wird.

5. Vorrichtung (1) nach Anspruch 3 oder 4, wobei die Vorrichtung (1) derart ausgelegt ist, dass, wenn das mindestens eine Führungselement (12) nach der Rückzugsbewegung im Rest des Gehäuses (2, 2a, 2b, 21) in der dritten Position angeordnet wird und der Rest des Gehäuses (2, 2a, 2b, 21) von dem mindestens einen weichen Element (11) entfernt wurde, das mindestens eine Führungselement (12) nicht vom Rest des Gehäuses (2, 2a, 2b, 21) getrennt werden kann oder vom Rest des Gehäuses (2, 2a, 2b, 21) getrennt werden kann, ohne es oder ein fest damit verbundenes Halterelement (14) zu berühren.

6. Vorrichtung (1) nach einem der Ansprüche 2 bis 5, wobei das mindestens eine weiche Element (11) mindestens eine weiche Kanüle (11) umfasst und das mindestens eine Führungselement (12) mindestens eine Führungsnadel (12) umfasst, die sich durch den Flüssigkeitskanal der weichen Kanüle (11) erstreckt, um sie während des Einführens in das Gewebe vorübergehend zu versteifen.

7. Vorrichtung (1) nach einem der vorhergehenden Ansprüche, wobei die Vorrichtung (1) ferner Aktivierungsmittel (15a, 15b) zum Aktivieren der Antriebsmittel (8, 13) umfasst, und wobei die Aktivierungsmittel (15a, 15b) so ausgelegt sind, dass sie vom Benutzer mit einer Hand betätigt werden können.

8. Vorrichtung (1) nach einem der vorhergehenden Ansprüche, wobei in der ersten Position die Einführanordnung (6) im Wesentlichen senkrecht zu einer Ebene ausgerichtet ist, die durch die mindestens eine Kontaktfläche (3) gebildet wird, und die Einführbewegung eine lineare Bewegung entlang einer Achse senkrecht zu dieser Ebene ist.

9. Vorrichtung (1) nach Anspruch 8, wobei in einer Ausgangsposition die Einführanordnung (6) im Wesentlichen parallel zu einer durch die mindestens eine Kontaktfläche (3) gebildeten Ebene ausgerichtet ist und von der Eingangsposition in die erste Position geschwenkt werden muss, bevor die Einführbewegung ausgeführt werden kann.

10. Vorrichtung (1) nach Anspruch 7 und nach Anspruch 9, wobei die Vorrichtung (1) so ausgelegt ist, dass bei Betätigung der Aktivierungseinrichtung (15a, 15b) die Einführanordnung (6) automatisch von der Ausgangsposition in die erste Position bewegt wird.

11. Vorrichtung (1) nach einem der vorhergehenden Ansprüche, wobei die Vorrichtung (1) durch eine Einführvorrichtung, die das Gehäuse und die Antriebseinrichtung bereitstellt, durch einen Einführkopf, der die Einführanordnung bereitstellt, und durch eine Grundplatte, die durch den Einführkopf bereitgestellt wird oder separat vorgesehen ist, gebildet wird, wobei der Einführkopf in der Einführvorrichtung aufgenommen ist, um mittels der Einführvorrichtung auf den Körper des Patienten aufgebracht zu werden, und die Grundplatte an dem Gehäuse befestigt ist, um mit dem Gehäuse gegen die Haut des Körpers eines Patienten gedrückt zu werden, und wobei die Einführvorrichtung so konstruiert ist, dass sie nach dem Aufbringen des Einführkopfes und seiner Entfernung von der Grundplatte einen anderen Einführkopf zum Anwenden dessen an dem Körper eines Patienten aufnehmen kann.

## Revendications

1. Dispositif (1) pour l'insertion d'au moins un élément d'insertion (11, 12) comportant une pointe de piquage (9) sous la forme d'au moins une pointe de capteur ou d'au moins une canule de perfusion dans le tissu du corps d'un patient et pour la fixation de celui-ci à la peau du corps, comprenant :
- un boîtier (2, 2a, 2b, 10, 21) prévoyant au moins une face de contact (3) pour placer le dispositif (1) sur la peau du corps du patient, la face de contact (3) supportant une couche (4) de colle compatible avec la peau pour la fixer à la peau, laquelle couche (4) est en particulier couverte d'une couche protectrice amovible (5) ;
- un système d'insertion (6) disposé de manière mobile dans le boîtier (2, 2a, 2b, 10, 21) et comprenant l'au moins un élément d'insertion (11, 12) à insérer dans le tissu et un élément support (7) connecté fixement à au moins une partie (11) de l'au moins un élément d'insertion (11, 12) qui est destiné à rester pendant une période prolongée à l'intérieur du tissu du corps ; et
- un moyen d'entraînement (8) disposé dans le boîtier (2, 2a, 2b, 10, 21) pour effectuer un mouvement d'insertion du système d'insertion (6) depuis une première position dans laquelle la pointe de piquage (9) de l'au moins un élément d'insertion (11, 12) est reculée par rapport à la face de contact (3) dans une deuxième position dans laquelle la pointe de piquage (9) de l'au moins un élément d'insertion (11, 12) dépasse au-delà de la face de contact (3), de sorte que l'au moins un élément d'insertion (11, 12) est inséré dans le tissu du corps du patient lorsque le dispositif (1), pendant le mouvement d'insertion du système d'insertion (6), est placé par sa face de contact (3) sur ledit corps ;
- la face de contact (3) étant formée par une plaque de base (10) qui est fixée de manière à ce que, une fois que le mouvement d'insertion a été réalisé, elle soit détachable du reste du boîtier (2, 2a, 2b, 21) ;
- l'élément support (7) du système d'insertion (6) comprenant un moyen de couplage (23) par lequel il est couplé à la plaque de base (10) lorsque le système d'insertion (6) est amené par le mouvement d'insertion dans la deuxième position, **caractérisé en ce que**
- le moyen d'entraînement comprend au moins un élément de stockage d'énergie pour fournir de l'énergie d'entraînement pour le mouvement d'insertion, et
- qu'un moyen manuel de chargement d'énergie pour transférer du courant à l'au moins un moyen de stockage d'énergie comprend deux éléments de boîtier qui peuvent être poussés l'un dans l'autre ou l'un vers l'autre.

2. Dispositif (1) selon la revendication 1, dans lequel l'au moins un élément d'insertion (11, 12) du système d'insertion (6) comprend au moins un élément mou (11) destiné à être inséré dans le tissu et au moins un élément de guidage (12) pour raidir temporairement l'au moins un élément mou (11) pendant son insertion dans le tissu, lequel élément de guidage (12) forme la pointe de piquage (9) pour piquer la peau du corps du patient lors de l'insertion de l'au moins un élément d'insertion (11, 12) dans le tissu ;
- l'élément support (7) étant connecté fixement à l'au moins un élément mou (11) ; et
- lorsque, après le mouvement d'insertion du système d'insertion (6), l'au moins un élément mou (11) est couplé à la plaque de base (10) à l'aide du moyen de couplage de l'élément support (7), l'au moins un élément de guidage (12) est couplé au reste du boîtier (2, 2a, 2b, 21) de manière à ce que, lorsque le reste du boîtier (2, 2a, 2b, 21) est retiré de la plaque de base (10), l'au moins un élément de guidage (12) soit retenu dans le reste du boîtier (2, 2a, 2b, 21) et soit enlevé avec le reste du boîtier (2, 2a, 2b, 21).

3. Dispositif (1) selon la revendication 2, dans lequel le dispositif (1) comprend en outre un moyen d'entraînement (13) pour effectuer, après le mouvement d'insertion du système d'insertion (6), un mouvement de rétraction de l'au moins un élément de guidage (12) depuis la deuxième position vers une troisième position dans lequel sa pointe de piquage (9) est reculée par rapport à la face de contact (3), de sorte que, lorsque l'au moins un élément mou (11) est maintenu dans la deuxième position par l'élément support (7) couplé à la plaque de base (10), l'au moins un élément de guidage (12) soit séparé de l'au moins un élément mou (11).

4. Dispositif (1) selon la revendication 3, dans lequel le dispositif (1) est conçu de manière à ce que, une fois que le mouvement d'insertion du système d'insertion (6) a été réalisé, le mouvement de rétraction automatique de l'au moins un élément de guidage (12) soit réalisé.

5. Dispositif (1) selon la revendication 3 ou 4, le dispositif (1) étant conçu de manière à ce que, lorsque, après le mouvement de rétraction, l'au moins un élément de guidage (12) est disposé dans le reste du boîtier (2, 2a, 2b, 21) dans la troisième position et que le reste du boîtier (2, 2a, 2b, 21) a été retiré de l'au moins un élément mou (11), l'au moins un élément de guidage (12) ne puisse pas être séparé du reste du boîtier (2, 2a, 2b, 21) ou puisse être séparé du reste du boîtier (2, 2a, 2b, 21) sans venir en contact avec lui ou avec un élément support (14) qui lui est connecté fixement.

6. Dispositif (1) selon l'une quelconque des revendications 2 à 5, dans lequel l'au moins un élément mou (11) comprend au moins une canule molle (11) et l'au moins un élément de guidage (12) comprend au moins une aiguille de guidage (12) qui s'étend à travers le canal à fluide de ladite canule molle (11) pour la raidir temporairement pendant l'introduction dans le tissu.

7. Dispositif (1) selon l'une quelconque des revendications précédentes, dans lequel le dispositif (1) comprend en outre un moyen d'activation (15a, 15b) pour activer le moyen d'entraînement (8, 13) et le moyen d'activation (15a, 15b) est conçu de manière à pouvoir être actionné d'une main par l'utilisateur.

8. Dispositif (1) selon l'une quelconque des revendications précédentes, dans lequel, dans la première position, le système d'insertion (6) est orienté substantiellement à la perpendiculaire d'un plan formé par l'au moins une face de contact (3) et le mouvement d'insertion est un mouvement linéaire le long d'un axe perpendiculaire audit plan.

9. Dispositif (1) selon la revendication 8, dans lequel, dans une position initiale, le système d'insertion (6) et orienté substantiellement parallèlement à un plan formé par l'au moins une face de contact (3) et, avant que le mouvement d'insertion puisse être réalisé, doit être pivoté de la position initiale selon vers la première position.

10. Dispositif (1) selon la revendication 7 et selon la revendication 9, dans lequel le dispositif (1) est conçu de manière à ce que, sur activation du moyen d'activation (15a, 15b), le système d'insertion (6) soit automatiquement passé de la position initiale à la première position.

11. Dispositif (1) selon l'une quelconque des revendications précédentes, dans lequel le dispositif (1) est formé par un dispositif d'insertion prévoyant le boîtier et le moyen d'entraînement, par une tête d'insertion prévoyant le système d'insertion et par une plaque de base prévue par la tête d'insertion ou prévue séparément, la tête d'insertion étant reçue dans le dispositif d'insertion pour être appliquée sur le corps du patient au moyen dudit dispositif d'insertion et la plaque de base étant fixée au boîtier afin d'être comprimée avec le boîtier contre la peau du corps d'un patient, et le dispositif d'insertion étant conçu de manière à ce que, après l'application de la tête d'insertion et son retrait de la plaque de base, il puisse recevoir une autre tête d'insertion pour l'appliquer sur le corps d'un patient.
